(19) Europäisches Patentamt
European Patent Office
Office européen des brevets

(11) Publication number: **0 165 710**
**A1**

(12) # EUROPEAN PATENT APPLICATION

(21) Application number: 85303496.5

(22) Date of filing: 17.05.85

(51) Int. Cl.[4]: C 07 C 101/62
C 07 C 103/82, C 07 C 93/20
C 07 C 143/24, A 61 K 7/06
A 61 K 7/44

(30) Priority: 21.06.84 US 623206

(43) Date of publication of application:
27.12.85 Bulletin 85/52

(84) Designated Contracting States:
DE FR GB IT

(71) Applicant: VAN DYK & COMPANY, INC.
Main and William Streets
Belleville New Jersey 07109(US)

(72) Inventor: Conner, Donald E.
76 Washington Avenue
Clifton New Jersey 07011(US)

(74) Representative: Bawden, Peter Charles
3 Maple Road
Harpenden Hertfordshire AL5 5TA(GB)

(54) **Benzene sulfonate quaternary ammonium salts of organic sunscreen carboxylic acids.**

(57) Compositions of benzene sulphonate quaternary ammonium salts of organic sunscreen carboxylic acids have substantivity for human hair and protect it from the undesirable effects of sunlight.

# BENZENE SULPHONATE QUATERNARY AMMONIUM SALTS OF ORGANIC SUNSCREEN CARBOXYLIC ACIDS

It has become increasingly apparent that UV light in addition to having detrimental effects on skin can also adversely affect human hair. Excessive exposure of hair to UV light causes brittleness, and in the case of dyed hair, discolouration, and loss of colour fidelity.

Organic sunscreens are generally unsuitable for application to human hair because of inadequate substantivity. Thus, they tend to rinse out of the hair and are accordingly ineffective.

Prior art attempts to remedy these shortcomings have not proved effective, or improved substantivity.

## Prior Art

US Patent 4,256,664 discloses halogen containing quats from amides of salicylic acids, but no tosylates. US Patent 4,061,730 discloses a tosylate quat of benzylidene camphor. US Patent 4,104,368 discloses cationic, anionic complexes formed by reacting an alkali metal salt of a sunscreen. French Patent 2,504,530 uses alkyl halides in the quaternisation step.

## Summary of the Invention

It has now been found that benzene sulphonate quaternary ammonium salt of organic sunscreen carboxylic acids, believed to be novel chemicals, have surprising substantivity for hair, and thus substantially protect it from the undesirable effects of sunlight.

## Detailed Description of the Invention

The quaternary salts of this invention correspond to the formula:

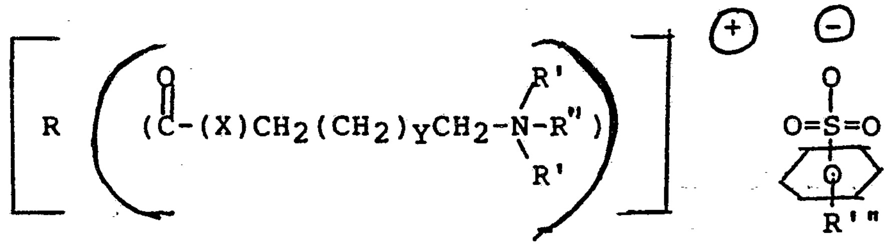

wherein the RCO moiety is selected from the group consisting of p-amino benzoic acid, p-dimethyl amino benzoic acid. p-methoxy cinnamic acid, p-methoxy benzal malonic acid, and cinnamal malonic acid; R' is an alkyl group having from1 to 2 carbon atoms; R" is an alkyl group having from 10 to 20 carbon atoms; R'" is selected from the group consisting of H and $CH_3$; X is selected from the group consisting of O and NH and Y is an integer of 0 to 1

Particularly preferred are those compounds where the RCO moiety is p-dimethyl amino benzoic acid, p-methoxy cinnamic acid, p-methoxy benzal malonic acid, or cinnamal malonic acid; where the X is -O-; where R" is $C_{16}$-$C_{18}$; and R' " is $CH_3$ and in the para position.

To obtain the derivative containing the necessary tertiary nitrogen various conventional techniques can be employed e.g. transesterification (of the methylester with a tert amino alcohol), reaction of an acid chloride with a tert amino alcohol using pyridine or triethylamine as solvent and catalyst; esterification of nitro benzoic acid with tert amino alcohols or amines, followed by reduction, or reductive alkylation, etc., e.g. see US Patent No. 4,102 398.

Then the derivative is quaternised by reaction, with an ester of the sulphonic acid The reaction is carried out conveniently in an isopropanol or toluene solution, which is heated to 75°-120° for about 3 to 6 hours.

The compounds of this invention are formulated into otherwise conventional compositions adapted for application to human hair. Thus they can be applied in an effective amount in cosmetic carriers known to the trade e.g. shampoos, sprays, and other speciality products. Concentrations of about 1 to 6 wt% in the composition are conveniently employed.

The compounds of this invention provide versatility in formulation since different compounds have different solubilities in water and organic solvents, and have peak absorptions at different pHs.

This invention, product work up, and properties of the materials will be better understood by reference to the following examples:

Example 1: cetyl stearyl sulphonate quaternary of 2-dimethylamino ethyl p-dimethylamino benzoate

Charge: 224g (1 mole) 2-dimethylamino ethyl-p-dimethylamino benzoate

422g (1 combining wt) cetyl/stearyl toluene sulphonate

600g toluene

The above materials were heated under reflux for 6 hours. At the end of this period, the mixture was cooled to room temperature and the solid filtered off. After recrystallisation twice from acetone (toluene can also be used) the solids were as charged. A yield of 565 grams (84.8% of theory) was obtained having a m.p. of 153°-157°C and an alkali number of 0.13.

Using the same procedure, the following compounds were prepared:

Example 2: lauryl p-toluene sulphonate quaternary of 2-dimethyl amino ethyl p-dimethyl amino benzoate

Off-white solid was obtained in 87% yield - alkali 2.6 and a m.p. of 133°-137°C.

Example 3: cetyl/stearyl p-toluene sulphonate quaternary of 2-dimethyl amino ethyl p-methoxy cinnamate

Compound obtained in 80% yield - melting point 139°-144°C and an alkali number of 0.0.

Example 4: cetyl/stearyl p-toluene sulphonate quaternary of 2-dimethyl amino ethyl p-dimethyl amino benzoate

Obtained in an 88% yield, melting point 106°-147°C, and alkali number of 1.6.

Example 5: di-cetyl/stearyl p-toluene sulphonate quaternary of di (2-dimethyl amino ethyl) p-methoxy benzal malonate

A yield of 89% was obtained, the compound had an alkali number of 3.5, and was a very viscous **liquid.**

Example 6

In order to demonstrate the substantivity and effectiveness of these compounds in prevention of the fading of dyed human hair, the following test programs were carried out on the compounds of Examples 3-5.

The test samples were prepared by taking 1 gram of dyed human hair in a 3% solution or dispersion of each of the test compounds. After standing for 1 hour the hair was removed from the solution, one half was air dried, and the other half was rinsed for 5 minutes in tap water and then dried.

All the test samples were irradiated with a Hanovia Ultra Violet Lamp containing a Corex D filter. The control was 2-ethylhexyl p-dimethylaminobenzoate. It was found that at 45 hours, fading in the control occurred with no fading of the treated sample.

The rinsed samples also retained 1/2 to 3/4 of their fade resistance.

Other materials of this invention work similarly. This work clearly demonstrates that these compounds are very good U.V. absorbers and that they have good substantivity for human hair, enough to prevent fading, superior to an ordinary comparable sunscreen.

Additional compounds for similar utility can be prepared utilising other organic sunscreens containing a carboxylic group.

The advantages of this invention will be apparent to those skilled in the art. Improved highly effective novel materials are provided having substantivity for hair which also impart protection against harmful sunlight.

It will be understood that this invention is not limited to the specific examples which have been offered as particular embodiments, and that modifications can be made without departing from the spirit thereof.

## CLAIMS

1 As a novel composition of matter a benzene sulphonate quaternary salt of an organic sunscreen carboxylic acid corresponding to the formula

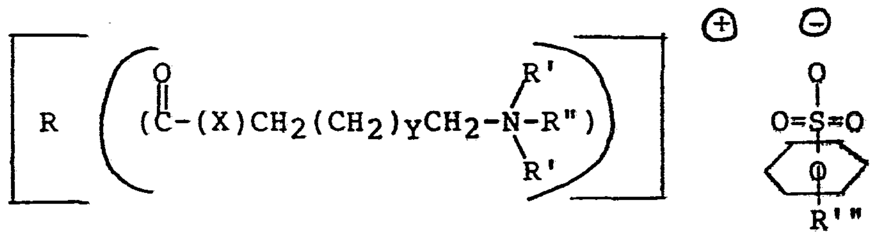

wherein the RCO moiety is selected from the group consisting of p-amino benzoic acid, p-dimethyl amino benzoic acid, p-methoxy cinnamic acid, p-methoxy benzal malonic acid, and cinnamal malonic acid; R' is an alkyl group having from 1 to 2 carbon atoms. R" is an alkyl group having from 10 to 20 carbon atoms. R'" is selected from the group consisting of H and $CH_3$; X is selected from the group consisting of 0 and NH and Y is an integer of 0 to 1.

2 The composition of Claim 1 in which the RCO moiety is selected from the group consisting of p-dimethyl amino benzoic acid, p-methoxy cinnamic acid, p-methoxy benzal malonic acid, and cinnamal malonic acid.

3 The composition of Claim 2 selected from the group consisting of cetyl/stearyl p-toluene sulphonate quaternary of 2-dimethyl amino ethyl p-dimethyl amino benzoate; cetyl/stearyl p-toluene sulphonate quaternary of 2-dimethyl amino ethyl p-methoxy cinnamate; and di-cetyl stearyl p-toluene sulphonate quaternary of di(2-dimethyl amino ethyl) p-methoxy benzal malonate.

4 A composition adapted for application to human hair comprising a cosmetic carrier containing an effective amount, sufficient to provide substantial protection from the effects of sunlight of a benzene sulphonate quaternary salt of an organic sunscreen carboxylic acid corresponding to the formula:

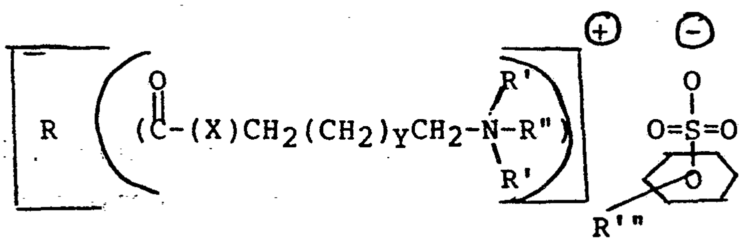

wherein the RCO moiety is selected from the group consisting of p-amino benzoic acid, p-dimethyl amino benzoic acid, p-methoxy cinnamic acid, p-methoxy benzal malonic acid, and cinnamal malinic acid; R' is an alkyl group having from 1 to 2 carbon atoms; R" is an alkyl group having from 10 to 20 carbon atoms; R'" is selected from the group consisting of H and $CH_3$; X is selected from the group consisting of O and NH and Y is an integer of 0 to 1.

5 The composition of Claim 4 in which the RCO moiety is selected from the group consisting of p-dimethyl amino benzoic acid, p-methoxy cinnamic acid, p-methoxy benzal malonic acid, and cinnamal malonic acid.

6 The composition of Claim 5 in which the salt is selected from the group consisting of cetyl/stearyl p-toluene sulphonate quaternary of 2-dimethyl amino ethyl p-dimethyl amino benzoate; cetyl/stearyl p-toluene sulphonate quaternary of 2-dimethyl amino ethyl p-methoxy cinnamate; and di-cetyl stearyl p-toluene sulphonate quaternary of di(2-dimethyl amino ethyl) p-methoxy benzal malonate.

7 A method of substantially protecting human hair from the effects of sunlight which comprises applying to the hair an effective amount of a benzene sulphonate quaternary salt of an organic sunscreen carboxylic acid corresponding to the formula:

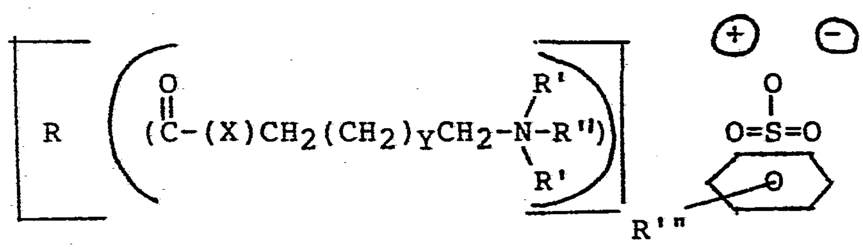

wherein the RCO moiety is selected from the group consisting of p-amino benzoic acid, p-dimethyl amino benzoic acid, p-methoxy cinnamic acid, p-methoxy benzal malonic acid, and cinnamal malonic acid; R' is an alkyl group having from 1 to 2 carbon atoms; R" is an alkyl group having from 10 to 20 carbon atoms; R'" is selected from the group consisting of O and NH and Y is an integer of 0 to 1.

8 The method of Claim 7 in which the RCO moiety is selected from the group consisting of p-dimethyl amino benzoic acid, p-methoxy cinnamic acid, p-methoxy benzal malonic acid, and cinnamal malonic acid.

9 The method of Claim 8 in which the salt is selected from the group consisting of cetyl/ stearyl p-toluene sulphonate quaternary of 2-dimethyl amino ethyl p-dimethyl amino benzoate; cetyl/stearyl p-toluene sulphonate quaternary of 2-dimethyl amino ethyl p-methoxy cinnamate; and di-cetyl stearyl p-toluene sulphonate quaternary of di(2-dimethyl amino ethyl) p-methoxy benzal malonate.

European Patent Office

# EUROPEAN SEARCH REPORT

0165710
Application number

EP 85303496.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE - A1 - 2 509 647 (GAF CORP.)<br>* Claims 1-4,13; page 8, line 20 - page 11 *<br>-- | 1-9 | C 07 C 101/62<br>C 07 C 103/82<br>C 07 C 93/20<br>C 07 C 143/24<br>A 61 K 7/06<br>A 61 K 7/44 |
| A | US - A - 3 403 207 (S.I. KREBS et al.)<br>* Claims *<br>-- | 1-9 | |
| D,A | FR - A - 2 504 530 (P. FABRE S.A.)<br>* Claims *<br>-- | 1-9 | |
| A | FR - A - 2 438 030 (KYOWA HAKKO KOGYO)<br>* Table 1 *<br>---- | 1,2 | |
| | | | TECHNICAL FIELDS SEARCHED (Int. Cl.4) |
| | | | C 07 C 93/00<br>C 07 C 101/00<br>C 07 C 103/00 |

The present search report has been drawn up for all claims

| Place of search | Date of completion of the search | Examiner |
|---|---|---|
| VIENNA | 01-08-1985 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document